# EUROPEAN PATENT SPECIFICATION

(11) **EP 0 391 369 B1**
(45) Date of publication and mention of the grant of the patent: **31.08.1994**
(21) Application number: 90106397.4
(22) Date of filing: 04.04.1990
(51) Int. Cl.: A61K 9/107, A61K 47/28

(54) **Medicinal emulsions**
Arzneiemulsionen
Emulsions pour médicaments

(30) Priority: 05.04.1989 IL 89856; 27.02.1990 IL 93558
(43) Date of publication of application: 10.10.1990
(73) Proprietor: YISSUM RESEARCH DEVELOPMENT COMPANY OF THE HEBREW UNIVERSITY OF JERUSALEM, Jerusalem, 92 182 (IL)
(72) Inventor: Simon, Benita, Jerusalem 90805 (IL); Menashe, Levy, Jerusalem 96181 (IL)
(74) Representative: VOSSIUS & PARTNER

(56) References cited:
- EP-A- 0 361 928
- WO-A-89/01327
- US-A- 4 115 313
- PATENT ABSTRACTS OF JAPAN, vol. 5, no. 200, 18th December 1981, page 102 C 84;& JP-A-56 122 309 (TEIJIN K.K.) 25-09-1981
- The Merck Index, 9th Edition, page 7349, Merck & Co., Inc., 1976
- The Merck Index, 10th Edition, page 7427, Merck & Co., Inc., 1983
- Lexikon der Pharmazie, page 653, Georg Thieme Verlag Stuttgart, 1987
- American Journal of Clinical Nutrition, Volume 50, pages 794-800, 1989 & Volume 52, pages 502-508, 1990
- Reallexikon der Medizin, M-R, page M71, Urban & Schwarzenberg, München 1977

## Description

The present invention concerns pharmaceutical compositions of hydrophobic drugs being in the form of an oil-in-water emulsion. The pharmaceutical compositions provided by the present invention show an outstanding long term stability and, in addition, in various forms of administration they also have sustained release characteristics. These pharmaceutical compositions when comprising a heat resistant drug are also remarkably stable to sterilisation by autoclaving.

Relevant prior art to the subject of the present invention may be found in the following publications:
1) U.S. Patent No. 3,172,816.
2) EP-A2-214501
3) EP-A3-215313.
4) Benita, S., Frieman, D. and Weinstock, M. (1986), International Journal of Pharmaceutics, 30: 47-55.
5) Singh, M. and Ravin, J. (1986), J. Parenteral Sci. Technol. 40: 34-41.
6) Von Dardel, O., Mebius, C. and Mossberg, T. (1976), Acta Anaesth. Scand. 20:221-224.

The utilization of pharmaceutical compositions in the form of emulsions of drugs having low aqueous solubility is well known in the art. However, as a rule such compositions are of low stability due to fast phase separation between the oil and the water phase, which is further enhanced by the hydrophobic drugs for which the emulsions serve as carriers.

Many hydrophobic drugs are important for various medical treatments, but since they are relatively unstable and insoluble in water their most useful form of administration is by way of an oil-in-water emulsion-type composition, in which the drug is dissolved in the oil phase. This is in effect the only practical way by which such hydrophobic drugs can be administered intravenously.

In accordance with the prior art stability of compositions of this type was unsatisfactory since as stated above, the hydrophobic drugs destabilize such compositions. Another drawback of prior art emulsion type compositions is that they tend to lose their stability when they are sterilized in an autoclave which is the most efficient and least costly way of sterilizing such compositions. During autoclaving the oily droplets of the emulsion coalesce and consequently creaming and/or phase separation occurs. This therefore necessitated hitherto the use of other forms of sterilization such as filtration as describes, for example, in EP-A2-214501 and EP-A3-215313.

US-A-4115313 discloses emulsions containing bile acids and fatty acid glycerides having 12-22 carbon atoms.

It is the object of the present invention to provide compositions of the oil-in-water type emulsions containing hydrophobic drugs, which are stable over prolonged storage and furthermore where the drug is heat resistant, are capable of being sterilized by autoclaving without a change in their properties or loss of their stability. It is a further object of the present invention to provide such compositions for parenteral, oral, ocular and topical administration of said drug.

In accordance with the invention it has surprisingly been found that oil-in-water type emulsions of a hydrophobic drug which comprise an oily carrier being a medium chain triglyceride (MCT) oil optionally in combination with vegetable oil, phospholipids, non-ionic surfactants and an ionic surfactant being a bile-duct surface active agent, cholic acid, or deoxycholic acid, or a surface active derivative or salt thereof are surprisingly more stable than prior art lipophilic drug-containing emulsions both in prolonged storage and in autoclaving.

The improved stability of the compositions in accordance with the invention was found to be caused by a syngergism which exists between the various ingredients, i.e. between said oily carrier, phospholipids, non ionic surfactant and said ionic surfactant.

In the following specification and claims all indication of percent (%) are by weight (w/v - weight of ingredient/volume of entire composition). All concentrations given below should be understood as standing each by itself and not cumulative.

The invention provides a pharmaceutical composition being an oil-in-water type emulsion comprising an effective amount of a hydrophobic drug, characterised in that it comprises about 3-50% of an oily carrier consisting of MCT oil optionally in combination with vegetable oil, about 0.05-20% of a phospholipid, about 0.03-10% of a non-ionic surfactant, and about 0.05-10% an ionic surfactant selected from the group consisting of ionic bile-duct surface active agent, cholic acid, and deoxycholic acid, and active derivatives and their salts.

An oil-in-water type emulsion generally comprises tiny oily droplets comprising the oily carrier (to be referred to hereinafter as the "oily phase") which are suspended in an aqueous solution (to be referred to hereinafter as "the aqueous phase"). The oily droplets are surrounded by a stabilizing interfacial film (to be referred to hereinafter for the sake of convenience as the "intermediate phase") formed by the phospholipids, the non-ionic surfactant and the said ionic surfactants.

MCT oil has many advantages over vegetable oil, amongst which are the following: lower susceptibility to oxidation; having a specific density of 0.94-0.95 which is higher than that of vegetable oil and which is close to that of water thus facilitating the obtaining of a stable emulsion; being less hydrophobic than vegetable oil and therefore enables achieving higher concentrations of the drug dissolved therein; having a lower viscosity which again enables obtaining a higher concentration of the oily phase in the composition without substantially increasing its viscosity.

On the other hand, vegetable oil has the advantage over MCT oil in its lower price. Thus, although the use of MCT oil by itself as the oily carrier is generally preferred in accordance with the invention, it may at times be practical to substitute some of it with vegetable oil.

Compositions in accordance with the present invention are suitable for topical, parenteral, ocular and oral administration of lipophilic drugs. Preferably the compositions are supplied in unit dosage form. Where a composition of the present invention is to be used for parenteral administration, it must be sterile, which sterilization is preferably performed by autoclaving. The ingredients in compositions to be used for parenteral administration will have to be of injection grade and medically approved for such administration.

An injectable composition should not be too viscous. As a rule, the viscosity of an emulsion increases, with an increase of the relative proportion of the non aqueous phase namely the oily and the intermediate phases, which comprise the oily carrier, phospholipids, non-ionic surfactant, said ionic surfactant and the hydrophobic drug. It is accordingly preferred, in accordance with the present invention, that the relative proportion of the non aqueous phase in injectable compositions, should not exceed about 30%. It is even more preferred, in accordance with the present invention, that the relative proportion of the non aqueous phase in injectable compositions be below about 25%.

On the other hand, topical compositions should preferably be viscous and to this end the relative proportion of the non-aqueous phase should preferably be above about 30%.

The preferred ranges of ingredients of injectable pharmaceutical compositions according to the invention are: oily carrier - about 10-20%; phospholipid - about 0.5-2%, 0.75%-2% being particularly preferred; non-ionic surfactant - about 3-10% or about 0.5-3%; said ionic surfactants - about 0.05-5% or about 0.3-10%, 0.5-2% being particularly preferred; drug - about 0.02-2%. In one embodiment, the relative amount of said ionic surfactants is about 4-6.6% when the amount of non-ionic surfactants is about 3-10%. Again these preferred ranges are to be understood as standing each by itself and are not cumulative.

The preferred pH of the aqueous phase of the composition of the invention is about 5.0-8.5, while 6.0-8.0 being more preferred, especially for parenteral administration.

Examples of MCT oil to be used in the compositions of the present invention are TCM (trade name - Societe des Oleagineaux, France), Miglyol 812 (trade name - Dynamit Nobel, Sweden).

Examples of vegetable oil which may be used in the compositions of the present invention are soybean oil, cotton seed oil, olive oil, and sesame oil.

Examples of phospholipids which may be used in compositions according to the invention are lecithins: EPIKURON or OVOTHIN 160 (trade names - both manufactured by Lucas Meyer, Germany) which are mixtures of mainly phosphatidylcholine and phosphatidylethanolamime from a natural source, such as purified egg yolk phospholipids (for the OVOTHIN series) and such as soybean oil phospholipids (for the EPIKURON series); a purified phospholipid mixture; Phospholipids E-80 (trade name - manufactured by Lipoid KG. Ludwigshafen, FRG).

An example of a non-ionic surfactant which may be used in compositions according to the invention is a poloxamer (PLURONIC F-68 BASF, Ludwigshafen, FRG), which is a copolymer of polyoxyethylene and polyoxypropylene.

Examples of said ionic surfactant to be used in compositions according to the invention are cholic acid and deoxycholic acid and surface active derivatives and salts thereof, which may be obtained commercially (Sigma, St. Louis, MO., U.S.A.). Preferred are cholic acid and dexoycholic acid and their sodium salts, deoxycholic acid and its sodium salt being particularly preferred.

The ionic surfactants used in accordance with the invention are anions of relatively weak acids and may thus associate with protons to become electrically neutral when the pH is reduced. Since these substances are surface active in their anionic state, the pH during preparations should be such in which the acid is dissociated into the anions end protons, i.e. the pH should have a higher value than the pKa (pKa - the pH in which half of the acid is dissociated). It was found however that after the composition had already been prepared, there was no reduction in stability even upon lowering of the pH.

The pharmaceutical compositions of the present invention preferably also comprise a preservative such as methyl-, ethyl-, propyl- and butylparaben which are medically accepted for parenteral administration. However, very often in accordance with the invention preservatives will not be required since the compositions may be sterilized by autoclaving without essentially reducing their stability. If desired, the pharmaceutical compositions of the present invention may also comprise an osmotic pressure regulator such as mannitol or glycerin, glycerin being preferred for parenteral administration and mannitol for oral administration. The compositions of the present invention may also comprise an antioxidant such as α-tocopherol.

The compositions of the present invention are suitable for the administration of hydrophobic drugs, i.e. drugs having a low water solubility. Examples of such drugs are: Hydrophobic or lipophilic antibiotic drugs such as amphotericin B; hydrophobic and lipophilic narcotic drugs such as alkaloid bases, e.g. morphine-base; hydrophobic benzodiazepines such as diazepam, fluphenazine deconoate and lorazepam; non-steroidal anti-inflammatory lipophilic drugs such as piroxicam and indomethacin; lipophilic steroids such as progesterone and testosterone propionate; lipophilic azoles such as miconazole and clotrimazole; lipophilic polypeptides such as cyclosporine; lipophilic sterols such as deoxycortone and calciferol; lipophilic cephalosporines; dimercaptol.

Where the drug in such compositions is amphotericin B, its concentration is preferably about 0.015 - 0.15%, about 0.075% being particularily preferred. Where the drug in said composition is morphine-base, its concentration is preferably about 0.2-2%. Where the drug in said composition is either diazepam or indomethacin, its concentration is preferably about 0.1-1%, about 0.4-0.5% being especially preferred. Where the drug in said composition is cyclosporine, its concentration is preferably about 2-5%. Where the drug in said composition is miconazole, its concentration is preferably about 1-3%.

Generally, the oily droplets in the oil-in-water emulsion for medical use should preferably be small, i.e. below about 1 µm, preferably below 0.5 µm and desirably below 0.2 µm, since the smaller the droplets, the more stable in storage is the emulsion. The droplet size is furthermore of particular importance if the emulsion is to be used for parenteral administration, and especially for intravenous injections, since large droplets will not readily pass through small blood capillaries. The compositions of the invention are particularly suitable for obtaining such small oily droplet.

The compositions of the present invention may be prepared by a number of ways. By one preparation mode, an aqueous solution and an oily solution are separately prepared, the aqueous solution comprising the non-ionic surfactant, the said ionic surfactant and the phospholipids and optionally also an osmotic pressure regulator and a preservative, and the oily solution comprising the said oily carrier, the hydrophobic drug and optionally also an anti-oxidant. By a slight modification of this mode, the aqueous solution is prepared from two a *priori* prepared solutions, a first, alcoholic, solution containing the said ionic surfactants and the phospholipid and a second solution containing in water the non-ionic surfactant and if desired also the other optional ingredients mentioned above. The said aqueous solution is then prepared by mixing the first and the second solution, then removing the alcohol, for sample by evaporation, to yield the said aqueous solution. This modified mode is suitable for the preparation of composition of the invention in which the drug is for example diazepam or miconazole and the like.

The aqueous solution and the oily solution are then mixed with one another. However, the so-obtained mixture does not yet consist of sufficiently small droplets, the size of which (obtained after mixing with a magnetic stirrer) is about 10 µm. The droplet size of the inventive composition may then be decreased by the use of emulsification equipment such as Ultra Turrax (Jankl and Kunkel, Staufen, FRG), which yields droplets having an average diameter of about 1.1 µm, or of a high shear mixer, e.g. Polytron (Kinematica, Lucerne, Switzerland) which yields droplets having an average diameter of about 0.65 µm.

Especially small droplets are obtained in the inventive compositions when utilizing a two-stage pressure homogenizer in which the crude dispersion is forced under high pressure through the annular space between a spring loaded valve and the valve seat, the second stage being in tandem with the first so that the emulsion is subjected to two very rapid dispersion processes. An example of such an apparatus is the Gaulin Homogenizer (APV Gaulin, Hilversum, The Netherlands). Use of such an apparatus in accordance with the invention yields compositions in which the droplets have an average diameter of about 0.27 µm with a relatively small deviation.

Even smaller droplets may be obtained in accordance with the invention when the emulsification process combines the use of both a Polytron-type high shear mixer followed by homogenization. The droplet size which is obtained in such a combination is about 0.1-0.15 µm. Achieving of such a droplet size is highly important because of the profound effect this has on the increase in the composition's stability and such is a result of both the ingredients used for the preparations of the composition, i.e. MCT oil and optionally vegetable oil, phospholipid, non-ionic surfactant and said ionic surfactant as well as the method of preparation as described above.

The present invention provides also a novel mode (to be referred to hereinafter as the "inventive mode") for the preparation of the above compositions. The inventive mode is particularly suitable for the preparation of compositions in accordance with the invention in which the drug is both hydrophobic and has a poor oil solubility, i.e. drugs which are present in the compositions predominantly in the intermediate phase. However, it should be noted that the inventive mode, as will be explained below, is principally suitable also for the preparation of compositions of the invention in which the hydrophobic drug is lipophilic, namely oil soluble and thus present in the compositions predominantly in the oily phase.

In accordance with the inventive mode the compositions are prepared by mixing together a liposome mixture and an oily mixture, each one prepared separately beforehand. The liposome mixture comprises all the ingredients which in the final composition do not form part of the oily phase, namely the phospholipids, the non-ionic surfactant, the said ionic surfactant, and, if desired, also the optional osmotic pressure regulator and the preservative. Where the drug has a poor oil solubility, such as Amphotericin B, it is also included in the liposome mixture. The preparation of the liposome mixture from these ingredients may be carried out by means known per se.

The oily mixture comprises the said oily carrier and, if desired, also the optional anti-oxidant. Where the drug is lipophilic, it is also included in the oily mixture.

After the lipasome mixture is mixed together with the oily mixture, an emulsion is formed having relatively large droplets, e.g. about 10 µm, which is further processed in a similar manner as described above in connection with the first preparation mode, until an emulsion having fine homogeneous droplets is obtained.

Compositions of the present invention which contain heat resistant drugs remarkably withstand sterilization in an autoclave. Such sterilization is easier to perform and more practical on industrial scales than sterilization by filtration as described in EP-A2 214501 and EP-A3-245313. However, where the drugs are heat sensitive, such as amphotericin B, standard aseptic conditions should be employed.

In the following Examples, the preparation of some specific compositions according to the present invention is described, it being understood that these Examples are for illustrative purposes only.

### DESCRIPTION OF THE DRAWINGS

In the following description, reference will at times be made to the annexed drawings in which:
Fig. 1 is a schematic representation of the process for extracting pure phospholipids from crude egg yolk phospholipids;
Fig. 2 is a schematic representation of an emulsion preparation process;
Fig. 3 shows droplet size distribution in a composition not containing any drug (a) and in a composition containing the drug amphotericin B (b);
Fig. 4 shows the droplet size distribution after three months storage at 4°C of compositions containing the drug amphotericin B (a) and compositions containing no lipophilic drug (b).
Fig. 5 shows the survival with time of *Candida albicans* infected mice after injection of Fungizone (trade name), an amphotericin B emulsion in accordance with the invention and of saline.

### EXPERIMENTAL METHODS

Measurement of emulsion properties in tests reported in some of the following Examples was performed as follows:

### A. Analysis of Particle Size

The droplet size distribution of the amphotericin B emulsions was determined by two complementary methods, namely by the photon correlation spectroscopy (PCS) using a computerized laser light scattering apparatus, considered the most appropriate for studying droplet size below 1 µm (Malvern system, Malvern, England), and the computerized laser inspection system which can measure droplet sizes above 0.6µm (Galai Cis-1, Migdal Haemek, Israel). In the Malvern system, each emulsion sample was diluted to the appropriate concentration with a filtered isotonic solution (2.25% glycerin in water) before measurement at 25°C. Duplicate samples were taken and each sample was analysed 10 times. In the Galai system, the samples were also diluted with 2.25% glycerin and two counts were made on each sample. The dilution was needed to increase viscosity thereby reducing the velocity movement of the droplets towards the surface.

The advantage of the Galai Cis-1 system over the widely used Coulter Counter system is demonstrated by the fact that there is no need for an electrolyte solution that can affect the stability of the emulsions. Both methods were needed since neither of them was able to measure accurately droplet sizes of the entire range of 100 to 3000nm. In most cases, the droplet size of the tested emulsions ranged from 100 to 400nm. However, when emulsions were subjected to accelerated stability tests, higher mean droplet size could have been in theory expected, and therefore it was necessary to measure diameters in the range of 0.6 to 2.0 µm. Thus, the droplet size distribution of emulsions which underwent stress conditions was analyzed using both the Galai Cis-1 and the Malvern systems.

### B. Measurements of Zeta Potential

The zeta potential was measured using the moving boundary electrophoresis technique, which has been shown to yield accurate electrophoretic mobility data. Measurement of electrophoretic mobility and conversion of electrophoretic mobility data to zeta potential, were performed similarly to that previously described (Benita et al., 1984, J.Pharm. Sci. 73: 1751-1755.). The electrolyte consisted of an aqueous solution containing 1% glycerol and 0.75% PLURONIC® F-68 which helped to stabilize the moving boundary (preventing the free diffusion of the droplets due to osmotic pressure which occurs in normal aqueous media) without altering the electrophoretic mobility.

Each emulsion sample was diluted with 9 pats of water to 1 part of sample, prior to examination. In order to confirm the measurements reliability various commercial Intralipid (trade name manufactured by Kabi-Vitrum) fat emulsions (which are emulsions used for nutrition, and comprise 10-20% soy oil, phospholipids and glycerin) were measured by this technique and were found to be identical, within the limits of experimental error, to corresponding zeta potential values published in the literature. Further confirmation of the reliability of this method came from the reproducibility of results.

### C. pH Measurement

The pH of the emulsion samples was measured using a pH meter (radiometer pH M63, Copenhagen, Denmark).

### Example 1: Preparation of an emulsion containing diazepam

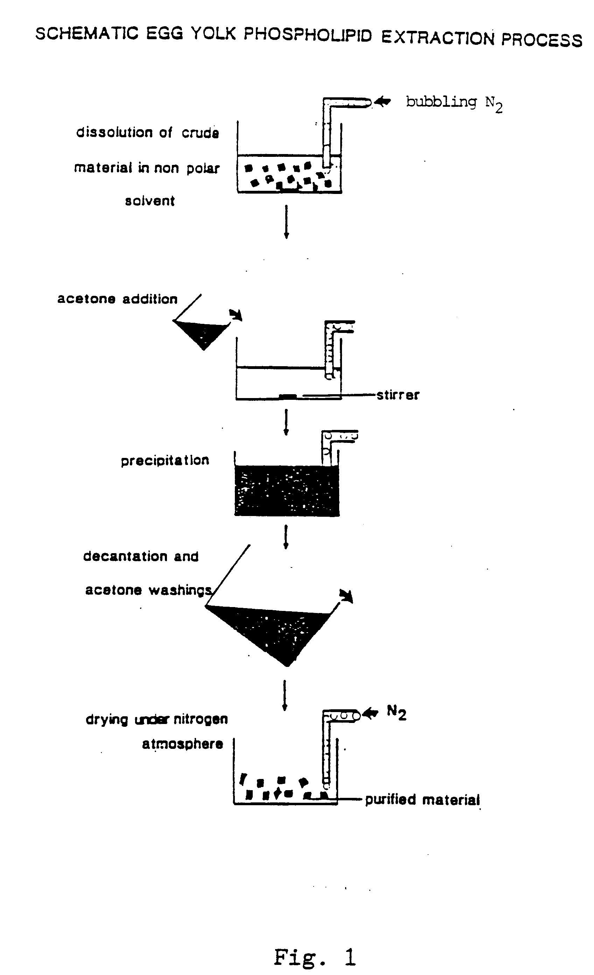

A) Oily, alcoholic and aqueous solutions were separately prepared as follows (all amounts below are given in % w/v in relation to the final total emulsion volume):
   I. Oily Solution:
      The oily solution consisted of the following ingredients:
      1) 10% TCM (Societe Industrielle de Oleagineaux, St. Laurent, Blangy, France), and 10% purified soybean oil (Bertin, Courbevoie, France), which was refrigerated for about a week to remove waxy substances.
      2) 0.05% α-tocopherol (Sigma Chemicals, St. Louis, MO, U.S.A.).
      3) 0.5% diazepam (Teva Pharmaceuticals Inc., Kfar Saba, Israel).
   II. Alcoholic Solution
      The alcoholic solution consisted of the following ingredients:
      1) 0.5% deoxycholic acid - D.C.A. (Sigma, St. Louis, MO, U.S.A.).
      2) 1.0% purified fractionated egg yolk phospholipids, prepared from crude egg yolk phospholipids (Sigma Chemicals, St. Louis, MO, U.S.A), in accordance with a modification of an earlier technique, reported by Schuberth and Wrethind (Acta. Chir. Scand. Suppl. 278: 1-21, 1961), as represented schematically in Fig. 1 of the annexed drawings. The purification generally consisted of four extraction cycles with petroleum ether 80-100 as extraction solvent. These purified phospholipids consisted mainly of phosphatidylcholine and phosphatidylethanolamine, together with small quantities of phosphatidylserine, phosphatidylinositol and phosphatidic acids (verified by standard TLC procedure).
   III. Aqueous Solution:
      The aqueous solution consisted of the following ingredients:
      1) 2% poloxamer PLURONIC® F-68, BASF, Ludwigshafen, FRG)
      2) 2.25% glycerin (Merck, Darmstadt, FRG).
      3) 0.2% methyl paraben (methyl p-hydroxybenzoic ester; Sigma Chemicals, St. Louis, MO., U.S.A.) and 0.075% of butyl paraben (butyl p-hydroxybenzoic ester; Sigma).
      4) Double distilled water (DDW) added up to 100%.
         Ingredient (3) was not always present and was only added when no autoclaving was to be applied.
         The alcohol solution was admixed with the aqueous solutions and the alcohol was removed by evaporation. The so obtained solution is the one which is hereinafter referred to as the "aqueous solution".

### Emulsion Preparation:

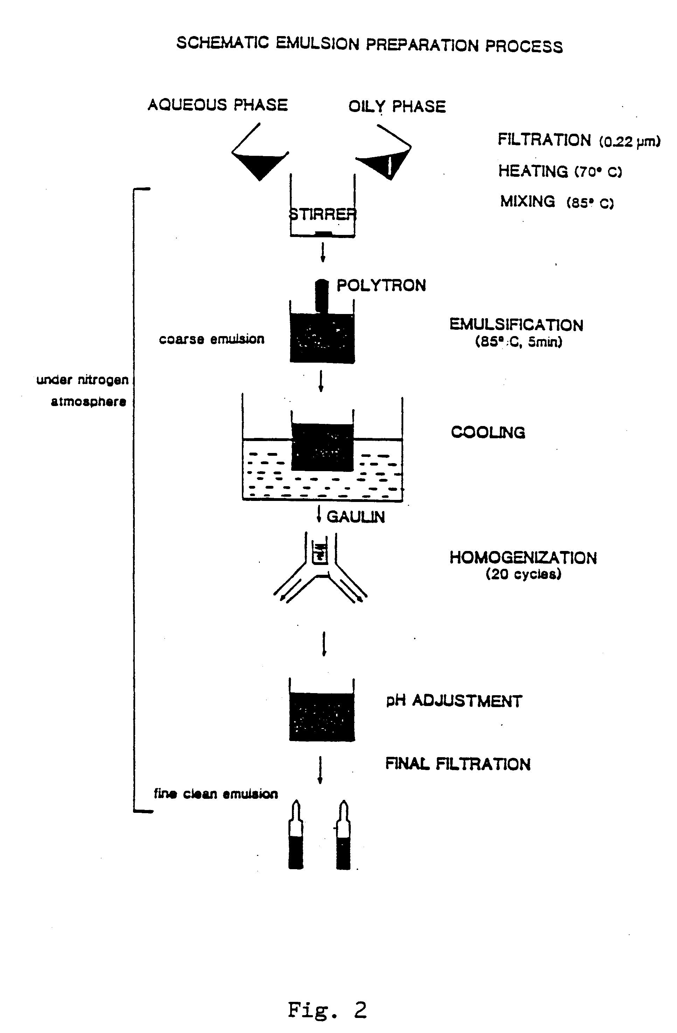

The whole process for the preparation of the emulsion was conducted under nitrogen atmosphere and under aseptic conditions. This process is schematically represented in Fig. 2 of the annexed drawings.

Both solutions were appropriately filtered using 0.22 µm Millipore (trade name) membrane filter. After filtration, the aqueous and the oily solutions were heated separately to 70°C and dispersed by a magnetic stirrer. Further heating was applied while mixing until the temperature reached 85°C. At this temperature, the two solutions ware mixed and emulsified during 5 min. using a high shear mixer, [Polytron (trade name) Kinematica, Lucerne, Switzerland]. The resulting coarse emulsion was cooled rapidly below 20°C. A fine monodispersed emulsion (i.e. having a small range of droplet sizes) was then achieved following 20 homogenization cycles, using a two-stage homogenizing valve assembly (Gaulin homogenizer, APV Gaulin, Hilversum, The Netherlands), at a temperature of 40-70°C under pressure of about 8500 psi.

Following the homogenization, the pH was adjusted to 8.0 using a 10% sodium hydroxide solution and the emulsion was filtered using a 1 µm filter, to discard the coarse droplets which ware generated during the emulsification and homogenization processes and also other debris.

Samples of the so filtered fine clean emulsion were stored in 10 ml brown ampoules and no phase separation was observed over periods of more than fourteen months.

### Example 2: Preparation of an emulsion containing diazepam

In a similar manner to that described in Example 1, diazepam containing emulsions were prepared, replacing, however, the purified fractionated egg yolk phospholipids (ingredient 3 of the oily solution) with OVOTHIN 160 (Lucas Meyer, Hamburg, Germany).

Similarly as in Example 1, no phase separation was seen even after storage of more than twelve months.

### Example 3: Stability of the emulsions

The stability of the emulsions of Examples 1 and 2 was determined by measuring various parameters of the emulsion at various time intervals following their preparation. The emulsions were stored at 4°C. Essentially no change in pH, zeta potential, mean droplet size, droplet size distribution and drug content was observed for more than fourteen months after preparing the emulsions. The stability tests are still under evaluation.

Some batches of the above emulsions were subjected, following their preparation, to a thermal shock by subjecting them to autoclaving - 1.1atm., 121°C, 15 min. Such a thermal shock is considered in the art as an effective accelerated test for assessing emulsion stability. However, surprisingly, even for such emulsions, no loss in stability was observed as determined by measuring the same above parameters. Similarly as above these emulsions retained their initial properties for more than four months after sterilization was performed.

### Example 4 : Stability of emulsions prepared with either deoxycholic acid or sodium deoxycholate

The emulsion of Example 2 was successively modified to give four different formulations as shown in the following Table I:

**TABLE I**

| Ingredients | Amount (%) | Formulations | | | |
|---|---|---|---|---|---|
| | | 1 | 2 | 3 | 4 |
| Soybean Oil | | - | 10.0 | - | 10.0 |
| MCT oil | | 20.0 | 10.0 | 20.0 | 10.0 |
| α-tocopherol | 0.02 | + | + | + | + |
| OVOTHIN 160 | 1.0 | + | + | + | + |
| Diazepam | 0.5 | + | + | + | + |
| PLURONIC® F-68 | 2.0 | + | + | + | + |
| SDC.* | | - | - | 1.0 | 1.0 |
| DCA.** | | 0.5 | 0.5 | - | - |
| Glycerol | .25 | + | + | + | + |
| Water to | 100 | + | + | + | + |

| | | | | | |
|---|---|---|---|---|---|
| * SDC. - Sodium deoxycholate | | | | | |
| ** DCA. - Deoxycholic acid | | | | | |

The zeta potential and the droplet size of the various formulations is shown in the following Table II:

**TABLE II**

| Formulation | Droplet size (nm) | | Zeta Potential (-mv) |
|---|---|---|---|
| | mean | S.D.* | |
| 1 | 112 | 27.9 | 48 |
| 2 | 117 | 29.8 | 45 |
| 3 | 125 | 31.2 | 35 |
| 4 | 137 | 36.8 | 32 |

| | | | |
|---|---|---|---|
| * S.D.- standard deviation | | | |

It can be seen that the compositions comprising MCT oil a the sole oily carrier had improved properties as compared to those prepared with equal concentrations of both MCT and vegetable oil. Additionally, the results show that emulsions comprising DCA had smaller, more uniform droplets and greater zeta potential than compositions comprising SDC. Hence the preference in utilizing DCA over SDC.

### Example 5 : Synergism between phospholipids, non-ionic surfactant and deoxycholic acid in diazepam containing emulsions

The emulsion of Example 2 (without α-tocopherol) was successively modified to give emulsions of different formulations a shown in Table III below:

**TABLE III**

| Ingredient | Concentration in % (w/v) | FI | FII | FIII | FIV | FV | FVI | FVII |
|---|---|---|---|---|---|---|---|---|
| Soybean Oil | 10.0 | + | + | + | + | + | + | + |
| MCT Oil | 10.0 | + | + | + | + | + | + | + |
| Diazepam | 0.5 | + | + | + | + | + | + | + |
| OVOTHIN 160 | 1.0 | + | + | + | - | + | - | - |
| PLURONIC® F-68 | 2.0 | + | + | - | + | - | + | - |
| Deoxycholic acid | 0.5 | + | - | + | + | - | - | + |
| Glycerin | 2.25 | + | + | + | + | + | + | + |
| Distilled water to 100.0 | | + | + | + | + | + | + | + |

All formulations were prepared on the same day with the same reagents and using the same preparative procedure, a described in Example 1. Following preparation the emulsions were sterilized by autoclaving (121° C, 1.1 atm, 15 min.).

The emulsions were first checked visually to determine the degree of creaming and those compositions that remained stable after 1 week were checked further to determine the droplet size and zeta potential.

The following results were obtained:

### Formulation FI:

This emulsion, which was the one of Example 1, was the most stable one and it remained stable for over fourteen months, and no creaming and phase separation were observed.

Zeta potential of this emulsion was measured to be -45mv and the mean droplet diameter was measured to be about 117 nm.

### Formulation FII:

The emulsion was unstable and 3-4 weeks after its preparation phase separation was observed. The phase separation was evident by the appearance of oil drops on the upper surface of the emulsion.

### Formulation FIII:

Phase separation was observed within one day after preparation of the emulsion.

### Formulation FIV:

Creaming was observed within a day of preparation of the emulsion. There was a smaller proportion of drops having a larger diameter, which caused creaming, which developed in the course of time to a total phase separation.

### Formulation FV:

The emulsion was unstable and total phase separation was observed within 2 days.

### Formulation FVI:

Creaming was observed in the emulsion within a day of its preparation. The degree of creaming increased in the following days resulting finally in a complete phase separation.

### Formulation VII

The emulsion was very unstable and phase separation was already observed in the course of its preparation.

### SUMMARY:

The emulsion of Formulation FI is the only one containing all the ingredients. The many-fold increase in stability over any of the emulsions of Formulations FII to FVII is attributed to the synergistic action which the oils, phospholipids, non ionic surfacants and deoxycholic acid have on long term stability of the composition.

### Example 6 : The effect of a thermal shock on a diazepam containing emulsions

The emulsions of Example 4 were subjected to a thermal shock by sterilizing in an autoclave (15 min, 121°, 1.180 atm). The mean droplet size (M.D.S.) and the standard deviation (S.D.) were determined before and after autoclaving and the results are given in the following Table IV:

**TABLE IV**

| Formulation No. | Before autoclaving | | After autoclaving | |
|---|---|---|---|---|
| | M.D.S. | S.D. | M.D.S. | S.D. |
| 1 | 112 | 27.9 | 112 | 29.6 |
| 2 | 117 | 29.8 | 126 | 31.2 |
| 3 | 125 | 31.2 | 150 | 39.4 |
| 4 | 137 | 36.8 | 135 | 37.6 |

The above results show clearly that sterilization had very little effect on droplet size of the emulsions and this remarkable result is in contrast to the instability of hitherto known emulsions of the oil-in water type.

### Example 7 : Stability to autoclaving of a miconazole base containing emulsions

In a similar manner to that described in Example 1 an emulsion comprising the following ingredients was prepared (% w/v):
Miconazole base - 1.0; MCT oil - 20.0; OVOTHIN 160 - 1.0; α-Tocopherol - 0.02; PLURONIC F-68 - 2.0; DCA - 0.5; Glycerol 2.25; and Water to 100.

The droplet size and standard deviation was measured before and after sterilization in an autoclave as in Example 6 and the results are shown in the following Table V:

**TABLE V**

| Before autoclaving | | After autoclaving | |
|---|---|---|---|
| M.D.S. | S.D. | M.D.S. | S.D. |
| 135 | 45.9 | 141 | 30.0 |

Here again, the surprising stability of the emulsion to autoclaving is demonstrated.

### Example 8: Preparation of an emulsion containing amphotericin B

Amphotericin B was dissolved in methanol (0.8 mg/ml) by bath sonication (15 min). Phospholipids E-80 (containing mainly 80% phosphatidylcholine and 8% phosphatidyl ethanolamine according to manufacturer specifications) were dissolved in chloroform. Both solutions were mixed and filtered through a combined filtering system comprising a fiber glass prefilter (GF. 92, Schleicher and Schuell, FRG) and 0.45µm regenerated cellulose membrane filter (RC 5, Schleicher and Schuell, FRG), for removing pyrogens and aggregates. The resulting clear lipid solution was deposited as a thin film on the walls of a round-bottom flask by rotary evaporation under reduced pressure at 40°C. The aqueous phase comprising the poloxamer, sodium deoxycholate and glycerin was filtered through a 0.22 µm Millipore (trade name, manufactured by Millipore, Bedford, Mass., U.S.A.) filter, poured into the flask and the dispersion was sonicated until a homogeneous liposomal mixture was achieved.

MCT oil, filtered through 0.22 µm Millipore filter and containing α-tocopherol was heated to 70°C and then admixed into the liposomal mixture heated to 45°C and dispersed therein by a magnetic stirrer. Emulsification was carried out while maintaining the same temperature using a high shear mixer, Polytron (Kinematica, Luzern, Switzerland). The resulting coarse emulsion was cooled rapidly. A fine monodispersed emulsion was achieved using a two stage homogenizer (APV Gaulin, Hilversum, The Netherlands).

Finally, the pH of the emulsion was adjusted to 8.0 with unbuffered 10% sodium hydroxide solution and the final emulsion was filtered through a 0.45 µm Millipore filter to discard coarse droplets and debris generated during the emulsification and homogenization processes.

All the processing operations were carried out under aseptic conditions. The sterility of the emulsions was assessed using the Bactec 46 apparatus (Johnson Laboratories, Towson, MD). This instrument is used to test inoculated Bactec culture vials for the presence of radioactive Carbon dioxide (¹⁴CO₂) in the vials. Should a high level of ¹⁴CO₂ be present in vials used for culturing aerobic or anaerobic organisms, it indicates that there were viable microorganisms in the original inoculum. The negative results obtained using this technique showed that the emulsions were sterile.

The relative amounts of the various ingredients in the final emulsions were as follows:
0.075% amphotericin B, 20% MCT oil 0 5% phospholipid E 80, 2.0% poloxamer, 1.0% sodium deoxycholate, 2.25% glycerine, 0.02% α-Tocopherol and bi-distilled water 200%.

For the purpose of various comparative tests to be reported below, also a plain emulsion, namely one which did not contain any lipophilic drug was prepared under identical experimental conditions.

### Example 9: Evaluation of the properties of an amphotericin B containing emulsion

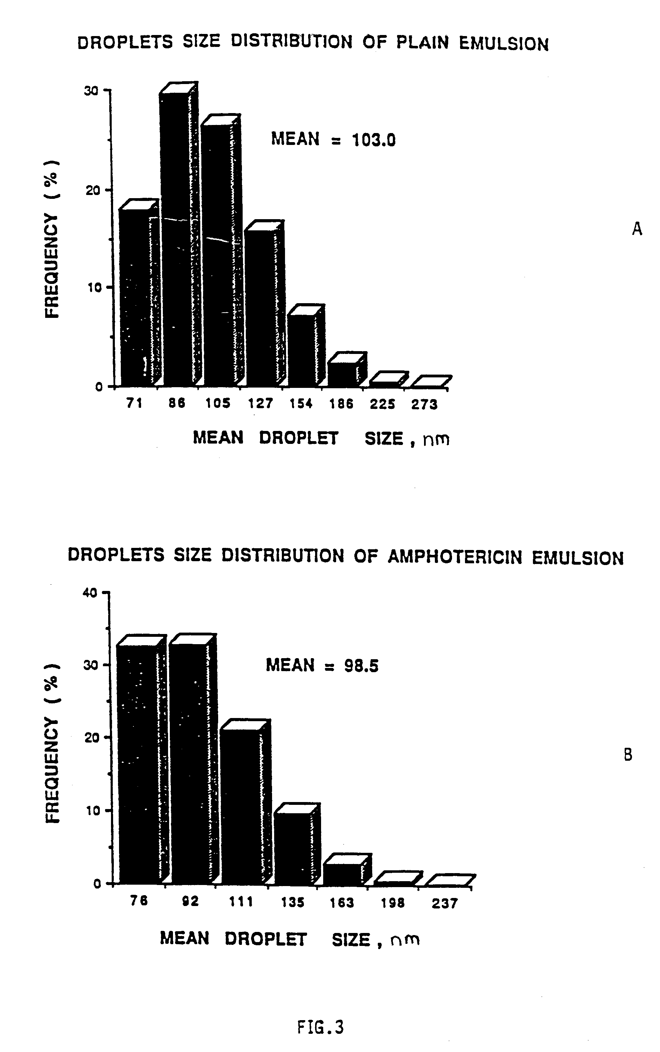

The emulsion of Example 8 was evaluated and found to have a mean droplet size of about 100 nm as shown in Fig. 3. Furthermore, as may be seen in this Figure, no notable difference was found in the droplet size between the amphotericin and the plain emulsion.

This is further shown in the following Table VI:

**TABLE VI**

| EMULSIONS | DROPLET SIZE | | | ZETA POTENTIAL (mv) | pH |
|---|---|---|---|---|---|
| | MEAN | S.D.(*) | POLY D.(**) | | |
| PLAIN | 103 | 29.8 | 0.122 | -35.3 | 8.0 |
| WITH AMPHOTERICIN | 97.5 | 23.8 | 0.130 | -32.4 | 8.0 |

| | | | | | |
|---|---|---|---|---|---|
| (*) S.D. - Standard deviation | | | | | |
| (**) Poly D - polydispersity, a factor reflecting the homogeneity of the population | | | | | |

As may be seen from Table VI, the droplet size of the two emulsions were very homogeneous. Furthermore, there was also no notable difference in the zeta potential between the two emulsions.

The amphotericin emulsion of Example 8 was stored for a period of three months at 4°C. After such a storage period, the zeta potential, the pH as well as the particle size distribution of both types of emulsion remained practically unchanged. Furthermore, the amount of amphotericin, determined by HPLC as known per se, did not change for up to three months storage at 4°C, indicating that the stability of amphotericin was not affected by its incorporation in the emulsion.

The amphotericin emulsion was subjected to an accelerated shaking stability test, at a shaking rate of 100 strokes per minute at 25°C for forty eight hours. No droplets having diameter above 1µm were detected by the Galai Cis-1 system while a slight and insignificant increase in the droplet size of the emulsion was noted with the Malvern PCS method. These results indicate that the amphotericin emulsion remained stable in spite of the mechanical stress.

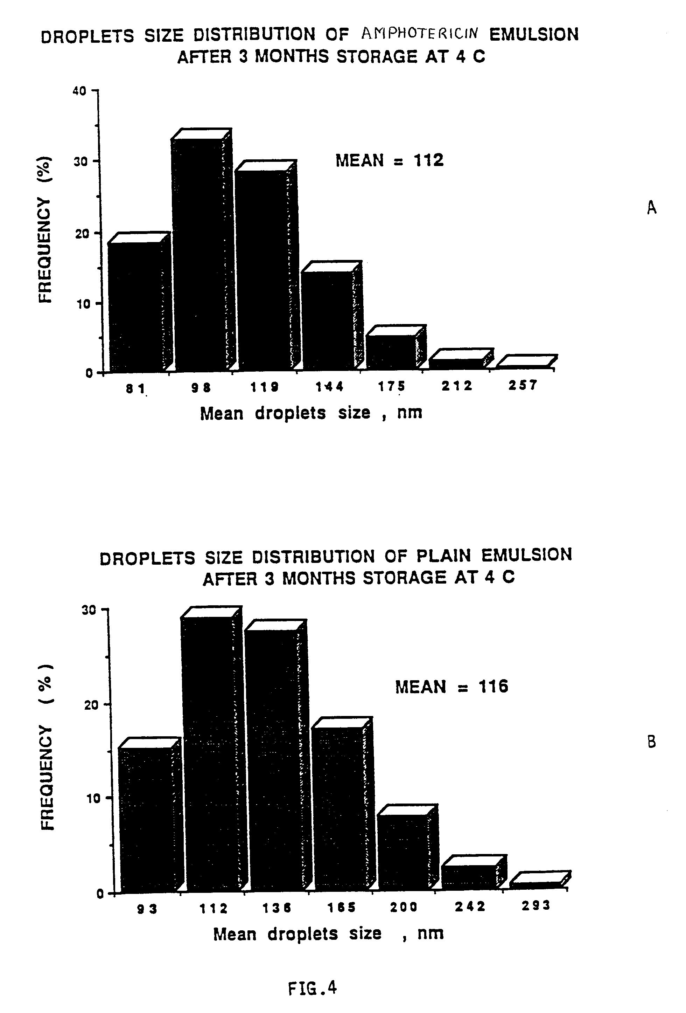

The droplet size distribution of amphotericin emulsion and plain emulsion after three months' storage at 4°C is shown in Fig. 4. It may be seen that there is no difference between the size distribution of the two emulsions and by comparing these results with those of Fig. 3, it may be seen that there is no substantial difference in the droplet size distribution after storage.

### Example 10: Animal studies with amphotericin formulations

Thirty Balb/C mice (weighing about 20g) were injected through the tail vein with 5 x 10⁵ of *Candida albicans,* strain 562, in 0.1 ml of saline. This dose was shown to cause the death of all the infected mice within five to ten days.

Forty eight hours after the infection was initiated each mouse received a post infection treatment. The mice were divided into three groups of ten mice, each group receiving a different treatment. The treatment consisted of an injection of 0.1 ml into the tail vein of one of the following formulations:
a) Fungizone which is a commercial amphotericin B formulation, (trade name manufactured by E.R. Squibb & Sons, Ltd.) at an amount of 0.4 mg/kg of amphotericin; proper dilution in order to achieve the aforementioned amount was achieved in accordance with manufacturers' specification;
b) An emulsion of Example 8, at an amount of 0.4 mg/kg; dilution of the batch formulation in order to achieve the aforementioned amount was performed with a "plain emulsion" (see Example 8);
c) Saline.

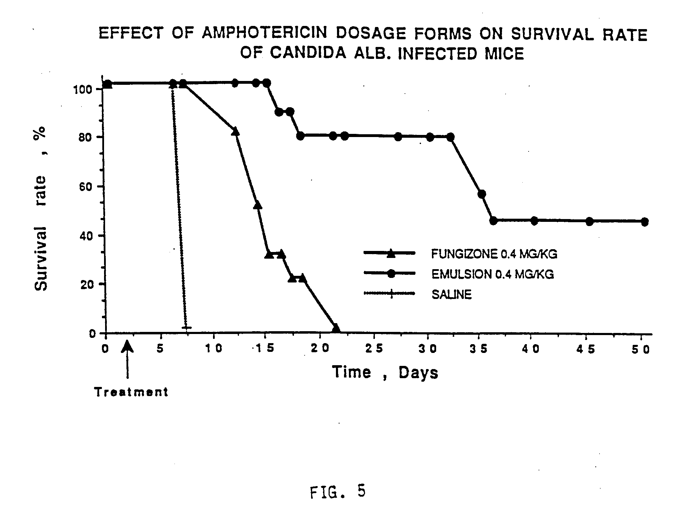

The number of surviving animals in each group was recorded daily, and the survival results are shown in Fig. 5.

It can be seen that all the untreated infected control animals died seven days after *Candida albicans* inoculation. All the infected mice which were treated with Fungizone died from infection 11-19 days after the injection while a much prolonged survival rate was observed in the animals injected with the amphotericin emulsions in accordance with the invention, and 50 days after inoculation, 55% of the mice were alive and appeared in a good condition.

These results demonstrate the improved medical properties of emulsions in accordance with the present invention.

Trademarks are acknowledged as such.

## Claims (Claims for the following Contracting State(s): AT, BE, CH, DE, DK, FR, GB, IT, LI, LU, NL, SE)

1. A pharmaceutical composition comprising an effective amount of a hydrophobic drug and a pharmaceutically acceptable carrier being an oil-in-water type emulsion comprising
(i) about 3-50% (w/v) of an oily carrier consisting of medium chain triglyceride (MCT) oil, optionally in combination with vegetable oil;
(ii) 0.05-20% (w/v) phospholipids;
(iii) about 0.03-10% (w/v) of a non-ionic surfactant; and
(iv) about 0.05-10% (w/v) of an ionic surfactant selected from bile-duct surface active agent, cholic acid and deoxycholic acid, and their surface active derivatives and salts.

2. A composition according to Claims 1 in a form suitable for parenteral administration.

3. A composition according to Claims 1 in a form suitable for topical administration.

4. A composition according to Claims 1 in a form suitable for oral adminisation.

5. A composition according to Claims 1 in a form suitable for ocular administration.

6. A composition according to Claim 2, in which the relative proportion of the non-aqueous phase which comprises said oily carrier, phospholipids, non-ionic surfactant and said ionic surfactant does not exceed 30%.

7. A composition according to Claim 6, in which the relative proportion of said non-aqueous phase does not exceed 25%.

8. A composition according to any one of the preceding claims, wherein the relative proportion of the oily carrier is about 10-20%.

9. A composition according to any one of the preceding claims, wherein the relative proportion of the phospholipid is about 0.5-2%.

10. A composition according to Claim 9, wherein the relative proportion of the phospholipid is about 0.75%.

11. A composition according to any one of the preceding claims, wherein the relative proportion of the non-ionic surfactant is about 0.5-3%.

12. A composition according to any one of the preceding claims, wherein the relative proportion of the said non-ionic surfactant is about 3%-10%.

13. A composition according to Claim 12, wherein the relative proportion of the said ionic surfactant is about 4-6.6%.

14. A composition according to any one of the preceding claims, wherein the vegetable oil is selected from soybean oil, cotton seed oil, olive oil, and sesame oil.

15. A composition according to any one of the preceding claims, wherein the non-ionic surfactant is a poloxamer.

16. A composition according to any one of the preceding claims, wherein the said ionic surfactant is selected from cholic acid, deoxycholic acid and their sodium salts.

17. A composition according to any one of the preceding claims, wherein said drug is selected from hydrophobic or lipophilic antibiotics or narcotic drugs, hydrophobic benzodiazepines, non-steroidal anti-inflammatory lipophilic drugs, lipophilic steroids, lipophilic azoles, lipophilic polypeptides, lipophilic sterols, lipophilic cephalosporines and dimercaptol.

18. A composition according to Claim 17, wherein said drug is selected from amphotericin B, morphine-base, diazepam, fluphenazine deconate, lorazepam, piroxicam, indomethacin, progesterone, testosterone propionate, miconazole, clotrimazole, cyclosporine, deoxycortone, calciferol, cephalosporine and dimercaptol.

19. A composition according to Claim 18, wherein said drug is morphine.

20. A composition according to any one of the preceding claims, wherein the mean oily droplet diameter is below 0.2µm.

21. A composition according to Claim 20, wherein the mean oily droplet diameter is about 0.1-0.15µm.

22. A process for the preparation of a composition of the oil-in-water type emulsion comprising an effective amount of a hydrophobic drug, about 3-50% (w/v) of an oily carrier consisting of MCT oil, optionally in combination with vegetable oil, about 0.05%-20% (w/v) of phospholipid, about 0.03-10% (w/v) of a non-ionic surfactant, and about 0.05-10% (w/v) of an ionic surfactant selected from bile-duct surface active agent, cholic acid and deoxycholic acid, and their surface active derivatives and salts, which process comprises:
(a) preparing a liposome mixture comprising the phospholipids, the non-ionic surfactant, the said ionic surfactant, and where the drug has a poor oil solubility, also comprising said drug;
(b) preparing an oily mixture comprising said oily carrier, and where the drug is lipophilic, also comprising said drug;
(c) mixing said liposome mixture with said oily mixture, whereby said emulsion is obtained.

23. A process according to Claim 22, wherein the so obtained emulsion is subjected to further stages in which smaller droplets in the emulsion are obtained and/or the homogeneity of the droplets' size is increased.

24. A process according to Claim 22 or 23, wherein said drug has a poor oil solubility.

25. A process according to Claim 24, wherein said drug is amphotericin B.

## Claims (Claims for the following Contracting State(s): ES, GR)

1. A process for the preparation of a composition of the oil-in-water type emulsion comprising an effective amount of a hydrophobic drug, about 3 to 50% (w/v) of an oily carrier consisting of medium chain triglyceride (MCT) oil, optionally in combination with vegetable oil, about 0.05 to 20% (w/v) of phospholipid, about 0.03 to 10% (w/v) of a non-ionic surfactant, and about 0.05 to 10% (w/v) of an ionic surfactant selected from bile-duct surface active agents, cholic acid and deoxycholic acid, and their surface active derivatives and salts, which process comprises:
(a) preparing a liposome mixture comprising the phospholipids, the non-ionic surfactant, the said ionic surfactant, and where the drug has a poor oil solubility, also comprising said drug;
(b) preparing an oily mixture comprising said oily carrier, and where the drug is lipophilic, also comprising said drug;
(c) mixing said liposome mixture with said oily mixture, whereby said emulsion is obtained.

2. A process according to Claim 1, wherein the so obtained emulsion is subjected to further stages, in which smaller droplets in the emulsion are obtained and/or the homogeneity of the droplets' size is increased.

3. A process according to Claim 1 or 2, wherein said drug has a poor oil solubility.

4. A process according to any one of Claims 1 to 3 in a form suitable for parenteral administration.

5. A process according to any one of Claims 1 to 3 in a form suitable for topical administration.

6. A process according to any one of Claims 1 to 3 in a form suitable for oral administration.

7. A process according to any one of Claims 1 to 3 in a form suitable for ocular administration.

8. A process according to Claim 4, in which the relative proportion of the non-aqueous phase which comprises said oily carrier, phospholipids, non-ionic surfactant and said ionic surfactant does not exceed 30%.

9. A process according to Claim 8, in which the relative proportion of said non-aqueous phase does not exceed 25%.

10. A process according to any one of the preceding claims, wherein the relative proportion of the oily carrier is about 10 to 20%

11. A process according to any one of the preceding claims, wherein the relative proportion of the phospholipid is about 0.5 to 2%.

12. A process according to Claim 11, wherein the relative proportion of the phospholipid is about 0.75%.

13. A process according to any one of the preceding claims, wherein the relative proportion of the non-ionic surfactant is about 0.5 to 3%.

14. A process according to any one of the preceding claims, wherein the relative proportion of the said non-ionic surfactant is about 3 to 10%.

15. A process according to Claim 14, wherein the relative proportion of the said ionic surfactant is about 4 to 6.6%.

16. A process according to any one of Claims 1 and 4 to 15, wherein the vegetable oil is selected from soybean oil, cotton seed oil, olive oil and sesame oil.

17. A process according to any one of the preceding claims, wherein the non-ionic surfactant is a poloxamer.

18. A process according to any one of the preceding claims, wherein the said ionic surfactant is selected from cholic acid, deoxycholic acid and their sodium salts.

19. A process according to any one of the preceding claims, wherein said drug is selected from hydrophobic or lipophilic antibiotics or narcotic drugs, hydrophobic benzodiazepines, non-steroidal anti-inflammatory lipophilic drugs, lipophilic steroids, lipophilic azoles, lipophilic polypeptides, lipophilic sterols, lipophilic cephalosporines and dimercaptol.

20. A process according to Claim 19, wherein said drug is selected from amphotericin B, morphine-base, diazepam, fluphenazine deconate, lorazepam, piroxicam, indomethacin, progesterone, testosterone propionate, miconazole, clotrimazole, cyclosporine, deoxycortone, calciferol, cephalosporine and dimercaptol.

21. A process according to Claim 20, wherein said drug is morphine.

22. A process according to any one of the preceding claims, wherein the mean oily droplet diameter is below 0.2 µm.

23. A process according to Claim 22, wherein the mean oily droplet diameter is about 0.1 to 0.15 µm.

## Patentansprüche (Patentansprüche für folgende(n) Vertragsstaat(en): AT, BE, CH, DE, DK, FR, GB, IT, LI, LU, NL, SE)

1. Arzneimittel, umfassend eine wirksame Menge eines hydrophoben Arzneistoffes und eines pharmazeutisch verträglichen Trägers, das eine Emulsion vom Öl-in-Wasser-Typ ist, umfassend
(i) etwa 3-50 % (w/v) eines Ölträgers, bestehend aus einem Triglyceridöl mit mittlerer Kettenlänge (MCT-Öl), gegebenenfalls in Kombination mit einem pflanzlichen Öl;
(ii) 0,05-20 % (w/v) Phospholipide;
(iii) etwa 0,03-10 % (w/v) eines nicht-ionischen Netzmittels; und
(iv) etwa 0,05-10 % (w/v) eines ionischen Netzmittels, ausgewählt aus einem oberflächenaktiven Mittel des Gallengangs, Cholsäure und Deoxycholsäure und ihren oberflächenaktiven Derivaten und Salzen.

2. Mittel gemäß Anspruch 1 in einer zur parenteralen Verabreichung geeigneten Form.

3. Mittel gemäß Anspruch 1 in einer zur lokalen Verabreichung geeigneten Form.

4. Mittel gemäß Anspruch 1 in einer zur oralen Verabreichung geeigneten Form.

5. Mittel gemäß Anspruch 1 in einer zur okularen Verabreichung geeigneten Form.

6. Mittel gemäß Anspruch 2, wobei der relative Anteil der nicht-wäßrigen Phase, die den Ölträger, Phospholipide, das nichtionische Netzmittel und das ionische Netzmittel umfaßt, 30 % nicht übersteigt.

7. Mittel gemäß Anspruch 6, wobei der relative Anteil der nicht-wäßrigen Phase 25 % nicht übersteigt.

8. Mittel gemäß einem der vorstehenden Ansprüche, wobei der relative Anteil des Ölträgers etwa 10-20 % ist.

9. Mittel gemäß einem der vorstehenden Ansprüche, wobei der relative Anteil des Phospholipids etwa 0,5-2 % ist.

10. Mittel gemäß Anspruch 9, wobei der relative Anteil des Phospholipids etwa 0,75 % ist.

11. Mittel gemäß einem der vorstehenden Ansprüche, wobei der relative Anteil des nicht-ionischen Netzmittels etwa 0,5-3 % ist.

12. Mittel gemäß einem der vorstehenden Ansprüche, wobei der relative Anteil des nicht-ionischen Netzmittels etwa 3-10 % ist.

13. Mittel gemäß Anspruch 12, wobei der relative Anteil des ionischen Netzmittels etwa 4-6,6 % ist.

14. Mittel gemäß einem der vorstehenden Ansprüche, wobei das pflanzliche Öl aus Sojabohnenöl, Baumwollsamenöl, Olivenöl und Sesamöl ausgewählt ist.

15. Mittel gemäß einem der vorstehenden Ansprüche, wobei das nicht-ionische Netzmittel ein Poloxamer ist.

16. Mittel gemäß einem der vorstehenden Ansprüche, wobei das ionische Netzmittel aus Cholsäure, Deoxycholsäure und ihren Natriumsalzen ausgewählt ist.

17. Mittel gemäß einem der vorstehenden Ansprüche, wobei der Arzneistoff aus hydrophoben oder lipophilen Antibiotika oder narkotischen Arzneistoffen, hydrophoben Benzodiazepinen, nicht-steroiden, entzündungshemmenden, lipophilen Arzneistoffen, lipophilen Steroiden, lipophilen Azolen, lipophilen Polypeptiden, lipophilen Sterolen, lipophilen Cephalosporinen und Dimercaptol ausgewählt ist.

18. Mittel gemäß Anspruch 17, wobei der Arzneistoff aus Amphotericin B, Morphin-Base, Diazepam, Fluphenazindecanoat, Lorazepam, Piroxicam, Indomethacin, Progesteron, Testosteronpropionat, Miconazol, Clotrimazol, Cyclosporin, Deoxycorton, Calciferol, Cephalosporin und Dimercaptol ausgewählt ist.

19. Mittel gemäß Anspruch 18, wobei der Arzneistoff Morphin ist.

20. Mittel gemäß einem der vorstehenden Ansprüche, wobei der mittlere Durchmesser der Öltröpfchen kleiner als 0,2 µm ist.

21. Mittel gemäß Anspruch 20, wobei der mittlere Durchmesser der Öltröpfchen etwa 0,1-0,15 µm ist.

22. Verfahren zur Herstellung eines Mittels aus einer Emulsion vom Öl-in-Wasser-Typ, umfassend eine wirksame Menge eines hydrophoben Arzneistoffes, etwa 3-50 % (w/v) eines Ölträgers, bestehend aus MCT-Öl, gegebenenfalls in Kombination mit einem pflanzlichen Öl, etwa 0,05-20 % (w/v) Phospholipid, etwa 0,03-10 % (w/v) eines nicht-ionischen Netzmittels und etwa 0,05-10 % (w/v) eines ionischen Netzmittels, ausgewählt aus einem oberflächenaktiven Mittel des Gallengangs, Cholsäure und Deoxycholsäure und ihren oberflächenaktiven Derivaten und Salzen, wobei das Verfahren umfaßt:
(a) Herstellung eines Liposomengemisches, umfassend die Phospholipide, das nicht-ionische Netzmittel, das ionische Netzmittel, und, wenn der Arzneistoff eine schlechte Öllöslichkeit aufweist, auch den Arzneistoff;
(b) Herstellung eines Ölgemisches, umfassend den Ölträger, und, wenn der Arzneistoff lipophil ist, auch den Arzneistoff;
(c) Mischen des Liposomengemisches mit dem Ölgemisch, wobei die Emulsion erhalten wird.

23. Verfahren gemäß Anspruch 22, wobei die erhaltene Emulsion weiteren Verfahrensschritten unterworfen wird, in denen die Tröpfchen in der Emulsion verkleinert werden, und/oder die Homogenität der Tröpfchengröße erhöht wird.

24. Verfahren gemäß Anspruch 22 oder 23, wobei der Arzneistoff eine schlechte Öllöslichkeit aufweist.

25. Verfahren gemäß Anspruch 24, wobei der Arzneistoff Amphotericin B ist.

## Patentansprüche (Patentansprüche für folgende(n) Vertragsstaat(en): ES, GR)

1. Verfahren zur Herstellung eines Mittels aus einer Emulsion vom Öl-in-Wasser-Typ, umfassend eine wirksame Menge eines hydrophoben Arzneistoffes, etwa 3-50 % (w/v) eines Ölträgers, bestehend aus einem Triglyceridöl mit mittlerer Kettenlänge (MCT-Öl), gegebenenfalls in Kombination mit einem pflanzlichen Öl, etwa 0,05-20 % (w/v) eines Phospholipids, etwa 0,03-10 % (w/v) eines nicht-ionischen Netzmittels und etwa 0,05-10 % (w/v) eines ionischen Netzmittels, ausgewählt aus oberflächenaktiven Mitteln des Gallengangs, Cholsäure und Deoxycholsäure und ihren oberflächenaktiven Derivaten und Salzen, wobei das Verfahren umfaßt:
(a) Herstellung eines Liposomengemisches, umfassend die Phospholipide, das nicht-ionische Netzmittel, das ionische Netzmittel, und, wenn der Arzneistoff eine schlechte Öllöslichkeit aufweist, auch den Arzneistoff;
(b) Herstellung eines Ölgemisches, umfassend den Ölträger, und, wenn der Arzneistoff lipophil ist, auch den Arzneistoff;
(c) Mischen des Liposomengemisches mit dem Ölgemisch, wobei die Emulsion erhalten wird.

2. Verfahren gemäß Anspruch 1, wobei die so erhaltene Emulsion weiteren Verfahrensschritten unterworfen wird, in denen die Tröpfchen in der Emulsion verkleinert werden, und/oder die Homogenität der Tröpfchengröße erhöht wird.

3. Verfahren gemäß Anspruch 1 oder 2, wobei der Arzneistoff eine schlechte Öllöslichkeit aufweist.

4. Verfahren gemäß einem der Ansprüche 1 bis 3 in einer für die parenterale Verabreichung geeigneten Form.

5. Verfahren gemäß einem der Ansprüche 1 bis 3 in einer für die lokale Verabreichung geeigneten Form.

6. Verfahren gemäß einem der Ansprüche 1 bis 3 in einer für die orale Verabreichung geeigneten Form.

7. Verfahren gemäß einem der Ansprüche 1 bis 3 in einer für die okulare Verabreichung geeigneten Form.

8. Verfahren gemäß Anspruch 4, in dem der relative Anteil der nicht-wäßrigen Phase, die den Ölträger, Phospholipide, das nichtionische Netzmittel und das ionische Netzmittel umfaßt, 30 % nicht übersteigt.

9. Verfahren gemäß Anspruch 8, in dem der relative Anteil der nicht-wäßrigen Phase 25 % nicht übersteigt.

10. Verfahren gemäß einem der vorstehenden Ansprüche, wobei der relative Anteil des Ölträgers etwa 10 bis 20 % ist.

11. Verfahren gemäß einem der vorstehenden Ansprüche, wobei der relative Anteil des Phospholipids etwa 0,5 bis 2 % ist.

12. Verfahren gemäß Anspruch 11, wobei der relative Anteil des Phospholipids etwa 0,75 % ist.

13. Verfahren gemäß einem der vorstehenden Ansprüche, wobei der relative Anteil des nicht-ionischen Netzmittels etwa 0,5 bis 3 % ist.

14. Verfahren gemäß einem der vorstehenden Ansprüche, wobei der relative Anteil des nicht-ionischen Netzmittels etwa 3 bis 10 % ist.

15. Verfahren gemäß Anspruch 14, wobei der relative Anteil des ionischen Netzmittels etwa 4 bis 6,6 % ist.

16. Verfahren gemäß einem der vorstehenden Ansprüche 1 und 4 bis 15, wobei das pflanzliche Öl aus Sojabohnenöl, Baumwollsamenöl, Olivenöl und Sesamöl ausgewählt ist.

17. Verfahren gemäß einem der vorstehenden Ansprüche, wobei das nicht-ionische Netzmittel ein Poloxamer ist.

18. Verfahren gemäß einem der vorstehenden Ansprüche, wobei das ionische Netzmittel aus Cholsäure, Deoxycholsäure und ihren Natriumsalzen ausgewählt ist.

19. Verfahren gemäß einem der vorstehenden Ansprüche, wobei der Arzneistoff aus hydrophoben oder lipophilen Antibiotika oder narkotischen Arzneistoffen, hydrophoben Benzodiazepinen, nicht-steroiden, entzündungshemmenden, lipophilen Arzneistoffen, lipophilen Steroiden, lipophilen Azolen, lipophilen Polypeptiden, lipophilen Sterolen, lipophilen Cephalosporinen und Dimercaptol ausgewählt ist.

20. Verfahren gemäß Anspruch 19, wobei der Arzneistoff aus Amphotericin B, Morphin-Base, Diazepam, Fluphenazindecanoat, Lorazepam, Piroxicam, Indomethacin, Progesteron, Testosteronpropionat, Miconazol, Clotrimazol, Cyclosporin, Deoxycorton, Calciferol, Cephalosporin und Dimercaptol ausgewählt ist.

21. Verfahren gemäß Anspruch 20, wobei der Arzneistoff Morphin ist.

22. Verfahren gemäß einem der vorstehenden Ansprüche, wobei der mittlere Durchmesser der Öltröpfchen kleiner als 0,2 µm ist.

23. Verfahren gemäß Anspruch 22, wobei der mittlere Durchmesser der Öltröpfchen etwa 0,1-0,15 µm ist.

## Revendications (Revendications pour l'(les) Etat(s) contractant(s) suivant(s): AT, BE, CH, DE, DK, FR, GB, IT, LI, LU, NL, SE)

1. Composition pharmaceutique comprenant une quantité efficace d'un médicament hydrophobe et d'un support pharmaceutiquement acceptable consistant en une émulsion du type huile dans eau comprenant (i) environ 3-50% (poids/volume) d'un support huileux consistant en une huile de triglycéride à chaîne moyenne (MCT), éventuellement en combinaison avec une huile végétale, (ii) 0,05-20% (poids/volume) de phospholipides, (iii) environ 0,03-10% (poids/volume) d'un agent tensioactif non ionique et (iv) environ 0,05-10% (poids/volume) d'un agent tensioactif ionique choisi parmi un agent tensioactif de canal biliaire, l'acide cholique et l'acide désoxycholique ainsi que parmi leurs dérivés tensioactifs et sels.

2. Composition suivant la revendication 1, sous une forme convenant pour l'administration parentérale.

3. Composition suivant la revendication 1, sous une forme convenant pour l'administration topique.

4. Composition suivant la revendication 1, sous une forme convenant pour l'administration orale.

5. Composition suivant la revendication 1, sous une forme convenant pour l'administration oculaire.

6. Composition suivant la revendication 2, dans laquelle la proportion relative de la phase non aqueuse qui comprend le support huileux, les phospholipides, l'agent tensioactif non ionique et l'agent tensioactif ionique n'excède pas 30%.

7. Composition suivant la revendication 6, dans laquelle la proportion relative de la phase non aqueuse n'excède pas 25%.

8. Composition suivant l'une quelconque des revendications précédentes, dans laquelle la proportion relative du support huileux est d'environ 10-20%.

9. Composition suivant l'une quelconque des revendications précédentes, dans laquelle la proportion relative du phospholipide est d'environ 0,5-2%.

10. Composition suivant la revendication 9, dans laquelle la proportion relative du phospholipide est d'environ 0,75%.

11. Composition suivant l'une quelconque des revendications précédentes, dans laquelle la proportion relative' de l'agent tensioactif non ionique est d'environ 0,5-3%.

12. Composition suivant l'une quelconque des revendications précédentes, dans laquelle la proportion relative de l'agent tensioactif non ionique est d'environ 3%-10%.

13. Composition suivant la revendication 12, dans laquelle la proportion relative de l'agent tensioactif ionique est d'environ 4-6,6%.

14. Composition suivant l'une quelconque des revendications précédentes, dans laquelle l'huile végétale est choisie parmi l'huile de soja, l'huile de graines de coton, l'huile d'olive et l'huile de sésame.

15. Composition suivant l'une quelconque des revendications précédentes, dans laquelle l'agent tensioactif non ionique est un poloxamère.

16. Composition suivant l'une quelconque des revendications précédentes, dans laquelle l'agent tensioactif ionique est choisi parmi l'acide cholique, l'acide désoxycholique et leurs sels de sodium.

17. Composition suivant l'une quelconque des revendications précédentes, dans laquelle le médicament est choisi parmi les antibiotiques ou médicaments narcotiques, hydrophobes ou lipophiles, les benzodiazépines hydrophobes, les médicaments lipophiles anti-inflammatoires non stéroïdiques, les stéroïdes lipophiles, les azoles lipophiles, les polypeptides lipophiles, les stérols lipophiles, les céphalosporines lipophiles et le dimercaptol.

18. Composition suivant la revendication 17, dans laquelle le médicament est choisi parmi l'amphotéricine B, la morphine base, le diazépam, le déconate de fluphénazine, le lorazépam, le piroxicam, l'indométhacine, la progestérone, le proprionate de testostérone, le miconazole, le clotrimazole, la cyclosporine, la désoxycortone, le calciférol, la céphalosporine et le dimercaptol.

19. Composition suivant la revendication 18, dans laquelle le médicament est la morphine.

20. Composition suivant l'une quelconque des revendications précédentes, dans laquelle le diamètre moyen des gouttelettes huileuses est en dessous de 0,2 µm.

21. Composition suivant la revendication 20, dans laquelle le diamètre moyen des gouttelettes huileuses est d'environ 0,1-0,15 µm.

22. Procédé pour la préparation d'une composition de l'émulsion du type huile dans l'eau comprenant une quantité efficace d'un médicament hydrophobe, environ 3-50% (poids/volume) d'un support huileux consistant en une huile de MCT, éventuellement en combinaison avec une huile végétale, environ 0,05%-20% (poids/volume) de phospholipide, environ 0,03-10% (poids/volume) d'un agent tensioactif non ionique et environ 0,05%-10% (poids/volume) d'un agent tensioactif ionique choisi parmi un agent tensioactif de canal biliaire, l'acide cholique et l'acide désoxycholique ainsi que parmi leurs dérivés tensioactifs et sels, lequel procédé comprend :
(a) la préparation d'un mélange liposomique comprenant les phospholipides, l'agent tensioactif non ionique, l'agent tensioactif ionique et dans lequel le médicament a une faible solubilité dans l'huile, comprenant également ledit médicament ;
(b) la préparation d'un mélange huileux comprenant le support huileux et dans lequel le médicament est lipophile, comprenant également ledit médicament ;
(c) le mélange du mélange liposomique avec le mélange huileux, de manière à obtenir l'émulsion susdite.

23. Procédé suivant la revendication 22, dans lequel l'émulsion ainsi obtenue est soumise à des stades ultérieurs dans lesquels les plus petites gouttelettes dans l'émulsion sont obtenues et/ou l'homogénéité de la taille des gouttelettes est accrue.

24. Procédé suivant l'une ou l'autre des revendications 22 et 23, dans lequel le médicament a une faible solubilité dans l'huile.

25. Procédé suivant la revendication 24, dans lequel le médicament est l'amphotéricine B.

## Revendications (Revendications pour l'(les) Etat(s) contractant(s) suivant(s): ES, GR)

1. Procédé pour la préparation d'une émulsion du type huile dans eau comprenant une quantité efficace d'un médicament hydrophobe, environ 3 à 50% (poids/volume) d'un support huileux consistant en une huile de triglycéride à chaîne moyenne (MCT), éventuellement en combinaison avec une huile végétale, environ 0,05 à 20% (poids/volume) de phospholipide, environ 0,03 à 10% (poids/volume) d'un agent tensioactif non ionique et environ 0,05 à 10% (poids/volume) d'un agent tensioactif ionique choisi parmi les agents tensioactifs de canaux biliaires, l'acide cholique et l'acide désoxycholique ainsi que parmi leurs dérivés tensioactifs et sels, lequel procédé comprend :
(a) la préparation d'un mélange liposomique comprenant les phospholipides, l'agent tensioactif non ionique, l'agent tensioactif ionique et dans lequel le médicament a une faible solubilité dans l'huile, comprenant également ledit médicament;
(b) la préparation d'un mélange huileux comprenant le support huileux et dans lequel le médicament est lipophile, comprenant également ledit médicament ;
(c) le mélange du mélange liposomique avec le mélange huileux, de manière à obtenir l'émulsion susdite.

2. Procédé suivant la revendication 1, dans lequel l'émulsion ainsi obtenue est soumise à des stades ultérieurs dans lesquels les plus petites gouttelettes dans l'émulsion sont obtenues et/ou l'homogénéité de la taille des gouttelettes est accrue.

3. Procédé suivant l'une ou l'autre des revendication 1 et 2, dans lequel les médicament a une faible solubilité dans l'huile.

4. Procédé suivant l'une quelconque des revendications 1 à 3, sous une forme convenant pour l'administration parentérale.

5. Procédé suivant l'une quelconque des revendications 1 à 3, sous une forme convenant pour l'administration topique.

6. Procédé suivant l'une quelconque des revendications 1 à 3, sous une forme convenant pour l'administration orale.

7. Procédé suivant l'une quelconque des revendications 1 à 3, sous une forme convenant pour l'administration oculaire.

8. Procédé suivant la revendication 4, dans lequel la proportion relative de la phase non aqueuse qui comprend le support huileux, les phospholipides, l'agent tensioactif non ionique et l'agent tensioactif ionique n'excède pas 30%.

9. Procédé suivant la revendication 8, dans lequel la proportion relative de la phase non aqueuse n'excède pas 25%.

10. Procédé suivant l'une quelconque des revendications précédentes, dans lequel la proportion relative du support huileux est d'environ 10 à 20 %.

11. Procédé suivant l'une quelconque des revendications précédentes, dans lequel la proportion relative du phospholipide est d'environ 0,5 à 2 %.

12. Procédé suivant la revendication 11, dans lequel la proportion relative du phospholipide est d'environ 0,75 %.

13. Procédé suivant l'une quelconque des revendications précédentes, dans lequel la proportion relative de l'agent tensioactif non ionique est d'environ 0,5 à 3 %.

14. Procédé suivant l'une quelconque des revendications précédentes, dans lequel la proportion relative de l'agent tensioactif non ionique est d'environ 3 à 10 %.

15. Procédé suivant la revendication 14, dans lequel la proportion relative de l'agent tensioactif ionique est d'environ 4 à 6,6 %.

16. Procédé suivant l'une quelconque des revendications 1 et 4 à 15, dans lequel l'huile végétale est choisie parmi l'huile de soja, l'huile de graines de coton, huile d'olive et l'huile de sésame.

17. Procédé suivant l'une quelconque des revendications précédentes, dans lequel l'agent tensioactif non ionique est un poloxamère.

18. Procédé suivant l'une quelconque des revendications précédentes, dans lequel l'agent tensioactif ionique est choisi parmi l'acide cholique, l'acide désoxycholique et leurs sels de sodium.

19. Procédé suivant l'une quelconque des revendications précédentes, dans lequel le médicament est choisi parmi les antibiotiques ou médicaments narcotiques hydrophobes ou lipophiles, les benzodiazépinez hydrophobes, les médicaments lipophiles anti-inflammatoires non stéroïdiques, les stéroïdes lipophiles, les azoles lipophiles, les polypeptides lipophiles, les stérols lipophiles, les céphalosporines lipophiles et le dimercaptol.

20. Procédé suivant la revendication 19, dans lequel le médicament est choisi parmi l'amphotéricine B, la morphine base, le diazépam, le déconate de fluphénazine, le lorazépam, le piroxicam, l'indométhacine, la progestérone, le proprionate de testostérone, le miconazole, le clotrimazole, la cyclosporine, la désoxycortone, le calciférol, la céphalosporine et le dimercaptol.

21. Procédé suivant la revendication 20, dans lequel le médicament est la morphine.

22. Procédé suivant l'une quelconque des revendications précédentes, dans lequel le diamètre moyen des gouttelettes huileuses est en dessous de 0,2 µm.

23. Procédé suivant la revendication 22, dans lequel le diamètre moyen des gouttelettes huileuses est d'environ 0,1 à 0,15 µm.
